# EUROPEAN PATENT APPLICATION

(11) **EP 3 974 377 A1**
(43) Date of publication of application: **30.03.2022**
(21) Application number: 20198904.3
(22) Date of filing: 29.09.2020
(51) Int. Cl.: B82Y 20/00, C12M 1/00, G01N 33/487, G02F 1/355, G02F 1/361, G03F 7/40

(54) **CELL CULTURE DEVICE AND METHOD FOR THERMAL MICRO-IRRADIATION OF CELLS**

(71) Applicant: Univerzita Palackého v Olomouci, 771 47 Olomouc (CZ)
(72) Inventor: Mistrik, Martin, 78301 Olomouc (CZ); Skrott, Zdenek, 77900 Olomouc (CZ); Bartek, Jiri, 2670 Greve (DK); Panacek, Ales, 78391 Unicov (CZ); Kvitek, Libor, 78372 Velky Tynec (CZ); Hochvaldova, Lucie, 79841 Kostelec na Hane (CZ)
(74) Representative: Hartvichova, Katerina

(57) **Abstract**

The present invention provides a cell culture device containing a substantially planar support, which further contains a polymeric film containing one or more polyelectrolytes provided on at least one surface of the support, and a plurality of plasmonic nanoparticles electrostatically bonded to the surface of the polymeric film.

Such device is useful for methods of thermal micro-irradiation of cells.

## Description

### Field of the Invention

The present invention relates to methods for preparing and use of cell culture devices allowing thermal micro-irradiation of selected cells or defined subcellular compartments, for example, using the standard laser-scanning microscope.

### Background Art

Exposition of cells and tissues to elevated temperatures is routinely used in the research of protein thermal stability profiling, thermal therapies, treatments of accidental burns, and proteinopathies involving the accumulation of defective proteins inside the cells. The cellular thermal damage primarily induces protein defects such as unfolding, aggregation, and denaturation, which are phenomena particularly crucial for the biology of illnesses such as Alzheimer's disease, Huntington disease, Parkinson's disease, amyotrophic lateral sclerosis (ALS), and amyloidosis. Accumulation of defective proteins also belongs to the hallmarks of cancer with the potential causative role, and cellular mechanisms responsible for the protein quality control belong to the anticancer therapeutic targets.

Studying responses to thermal damage of the proteins on the level of a single living cell or even on the subcellular level represents a significant challenge due to the lack of available systematic solutions allowing precise and fast delivery of the heat to the target structure at the micrometer scale. Currently, the temperature elevation is achieved by heating the cultivation media using various heat sources such as a water bath, an incubator with preset target temperature, or by various energy emitters, including microwaves, ultrasound, and infra-red lamps and lasers (Guin et al., PLOS ONE, 2019, https://doi.org/10.1371/journal.pone.0222990). However, such approaches have limitations. That includes a significant time delay in achieving the desired temperature as the cultivation media must be heated primarily, followed by heat transfer to the cells. Another restriction is spatial as the whole cultivation vessel, or at least its large part involving multiple cells is inevitably exposed to heating by such approaches.

Direct focusing of the heat to the individual cells or subcellular compartments within the micrometer scale is not achievable by any of the currently available approaches, which significantly limits the single-cell biology research options. Such localized, targeted heat damage at the subcellular level can be used in various research applications, including the studies of recruitment kinetics of proteins to the heat-damaged sites, differential response within individual compartments of the cell body, behavior and processing of heat-damaged proteins in the context of the cellular environment, and the transmission of stress signals from heat-damaged cells to the surrounding non-damaged cells (also known as a bystander effect).

### Summary of the Invention

The present invention provides a cell culture device which contains:
- a substantially planar support;
- a polymeric film containing one or more polyelectrolytes provided on at least one surface of the support;
- a plurality of plasmonic nanoparticles electrostatically bonded to the surface of the polymeric film.

The present invention further provides a method of preparation of the cell culture device, said method comprising the steps of:
- providing substantially planar support;
- applying a polymeric film containing one or more polyelectrolytes on at least one surface of the support;
- applying plasmonic nanoparticles onto the surface of the polymeric film.

The present invention also provides a method of thermal-microirradiation of cells, comprising the steps of:
- providing the cell culture device of the invention with a cell culture thereon,
- irradiating one or more plasmonic nanoparticles with an electromagnetic irradiation beam (e.g., a laser beam), including absorption frequency of the plasmonic nanoparticles, thereby locally heating the nanoparticle and the surroundings of the nanoparticle.

The present invention uses plasmonic nanoparticles for local heating at the level of an individual cell, or an individual cell organelle. Such localized heating cannot be achieved by the methods known in the art. Typically, the absorption frequencies of plasmonic nanoparticles are in the visible to near-infrared wavelengths/frequencies. The laser beam may irradiate a region of interest with a minimum size of approximately 200 nm².

The heating thus occurs at a scale ranging from hundreds of nanometers to units of millimeters, most typically at a micrometer scale (i.e., hundreds of nanometers to hundreds of micrometers). The surroundings of the irradiated nanoparticle, which is locally heated, is typically a region extending hundreds of nanometers to units of millimeters, preferably hundreds of nanometers to hundreds of micrometers, from the particle in all directions.

### Detailed description of the Invention

The cell culture device of the present invention contains support and polymeric film with a plurality of plasmonic nanoparticles attached to the polymeric film.

Preferably, the cell culture device containing the support, the polymeric film and the plurality of plasmon nanoparticles has a transparency of at least 50 %, more preferably 80 to 98 %, for electromagnetic irradiation having the wavelength of 550 nm. The transparency is measured in the direction perpendicular to the plane of the support. The transparency is needed in order to allow for observation of cells affected by the thermal-microirradiation.

The support is preferably made of a material which is substantially transparent for the laser beam. The surface of the material of the support may be charged (positively or negatively) or electroneutral in the air or in the liquid media. Such materials are preferably glass or transparent plastic, such as for example, polycarbonate, polymethylmethacrylate, polyethylene, polyethylene copolymers, polypropylene, polypropylene copolymers, polystyrene, polystyrene copolymers, styrene methyl methacrylate, poly(styrene-co-acrylonitrile), methyl methacrylate-acrylonitrile-butadiene-styrene, methyl methacrylate-butadiene-styrene, polyacetate, polyamide, polyethyleneterephtalate.
The support is substantially planar so that it can be used in laser-scanning procedures. Suitable supports include, for example, microscope slides, Petri dishes, or multi-well plates. Typically, the thickness of the support is in the range of 0.1 mm to 2 mm.

The polymeric film should be substantially transparent for the laser beam, inert, chemically stable, and cell-growth-compatible.
The individual polyelectrolytes present in the film are preferably positively charged polyelectrolytes and/or negatively charged polyelectrolytes. In some embodiments, a combination of at least one positively charged polyelectrolyte and at least one negatively charged polyelectrolyte is present in the film.
When the film contains more than one polyelectrolyte, the polyelectrolytes are preferably arranged in layers, each layer containing (predominantly) one polyelectrolyte. In some embodiments, two layers are present.
Each layer of the polyelectrolyte is typically a monomolecular layer. A monomolecular layer is a layer having the thickness of one molecule.
Such films are called in the art "thin polymeric films".
Suitable positively charged polyelectrolytes (polymers) may be, for example, poly(allylamine hydrochloride) (PAH), polyethyleneimine (PEI), chitosan, poly(diallyldimethylammonium chloride) (PDDA), poly-L-(lysine) (PLL), or poly(amidoamine) (PAM).
Suitable negatively charged polyelectrolytes (polymers) may be, for example, poly(vinyl sulfate) (PVS), poly(acrylic acid) (PAA), poly(beta-amino ester) (PBAE), poly(styrene sulfonate) (PSS), or poly(methacrylic acid) (PMA).
Preferable combinations of polyelectrolytes for the preparation of polymeric films are combinations of a positively charged polyelectrolyte and a negatively charged polyelectrolyte, such as:
- poly(acrylic acid) (PAA)/poly(allylamine hydrochloride) (PAH)
- poly(acrylic acid) (PAA)/ poly(diallyldimethylammonium chloride) (PDDA)
- poly(beta-amino ester) (PBAE)/chitosan
- poly(styrene sulfonate) (PSS)/poly(ethyleneimine)(PEI)
- poly(styrene sulfonate) (PSS)/ poly(diallyldimethylammonium chloride) (PDDA)
- poly(methacrylic acid) (PMA)/ poly(diallyldimethylammonium chloride) (PDDA)
- poly(methacrylic acid) (PMA)/ poly(ethyleneimine)(PEI)
- poly(methacrylic acid) (PMA)/Poly(amidoamine) (PAM)
- poly(beta-amino ester) (PBAE)/poly-L-(lysine) (PLL).

In embodiments with a single layer of polyelectrolyte, the polyelectrolyte should preferably be oppositely charged than the surface charge of the support, i.e., when the surface charge of the support is positive, a negatively charged polyelectrolyte layer will be applied, and vice versa. When the surface of the support is electroneutral, the suitable polymers to form the polyelectrolyte layer on the support are polymers enabling hydrogen bonding such as PAA. The plasmonic nanoparticles should have an opposite charge than the polyelectrolyte, to ensure electrostatic bonding.

In multi-layer embodiments of the polymeric film, the type of the first polyelectrolyte layer applied on the surface of the support depends on the chemical composition and surface charge of the support. The polymer should preferably be oppositely charged than the surface charge of the support, i.e., when the surface charge of the support is positive, a negatively charged polyelectrolyte layer will be applied, and vice versa. When the surface of the support is electroneutral, the suitable polymers to form the first polyelectrolyte layer on the support are polymers enabling hydrogen bonding such as PAA. In order to electrostatically bind plasmonic nanoparticles, the last polyelectrolyte layer (the layer onto which plasmonic nanoparticles are applied) should be oppositely charged than the surface charge of the plasmonic nanoparticles. Neighboring polyelectrolyte layers should have an opposite charge, i.e., when one layer has a positive surface charge, the neighboring polyelectrolyte layer should have a negative surface charge, and vice versa.

The term "oppositely charged" or "charged oppositely" means that the corresponding layer or component has an opposite electric surface charge than the other layer or component. This term is mostly used in connection with polyelectrolyte layers, or polyelectrolyte layers and support, or polyelectrolyte layers and plasmonic nanoparticles.

The maximum thickness of polymeric film with bonded plasmonic nanoparticles is such that the layer is still transparent for laser beam and preferably varies from tenths of nanometers to hundreds of micrometers, preferably from units to hundreds of nanometers, depending on the (i) chemical type and number of polyelectrolytes forming polyelectrolyte layer(s) and (ii) type, diameter, thickness and shape of plasmonic nanoparticles attached to the polymeric film. In some embodiments, the thickness of polymeric film with bonded plasmonic nanoparticles corresponds to the thickness of one polyelectrolyte layer (monomolecular layer) and to the type, diameter, thickness, and shape of electrostatically bonded plasmonic nanoparticles. In some embodiments, where multiple polyelectrolyte layers are present, the thickness of polymeric film with bonded plasmonic nanparticles corresponds to the sum of thicknesses of the polyelectrolyte layers (monomolecular layers) and to the type diameter, thickness, and shape of electrostatically bonded plasmonic nanoparticles.

The polymeric film can be prepared by applying the solution(s) of polyelectrolyte(s). When more than one polyelectrolyte is used, individual solutions are applied one after another, thus forming layers containing individual polyelectrolytes. Thus, the polymeric film can be formed on the surface of the support as a multi-layered film by repeated deposition of polyelectrolytes.

The surface of the support must be clean before the polymeric film is applied. If needed, the surface of the support is cleaned by suitable cleaning solutions, such as piranha solution, detergent and/or ethanol, and/or washed by distilled water. In order to prepare the polymeric film, a solution (preferably an aqueous solution) of a polyelectrolyte is applied on the surface of the support. Volume and concentration of the polyelectrolyte solution can typically vary from 0,001 ml to 500 ml and 0,001 % to 10 % (w/w), preferably from 0,01 ml to 100 ml and 0,01 % to 5 % (w/w) and more preferably from 0,1 ml to 50 ml and 0,1 % to 2 % (w/w), respectively. After application, molecules of polyelectrolytes dissolved in solution are left to bond electrostatically or through hydrogen bond or adsorbed, typically for the period of at least 30 min up to 5 hours, preferably from 60 min to 4 hours and more preferably from 90 min to 2 hours. After that, the polyelectrolyte solution is removed, and the surface of the support is washed by distilled water in order to remove un-bonded polyelectrolyte molecules. For multi-layer embodiments, second and further layers of polyelectrolytes are applied in the same way as the first layer, including the step of washing the surface of the support with the bound layers with distilled water between the application of individual layers.

Plasmonic nanoparticles are nanoparticles (NPs) having optical properties originating from the coherent oscillation of the conduction electrons upon exposure to light of specific wavelengths. When illuminated by light, free electrons localized on the nanoparticle surface become excited, and the local electron cloud is asymmetrically distributed over the whole nanoparticle. This distribution produces a coulombic restoring force between positively charged nuclei and negatively charged electrons from the conduction band, which leads to collective oscillation of the electron cloud on the particle surface called localized surface plasmon (LSP). The localized surface plasmon resonance (LSPR) takes place if the frequency of the incident light matches with the frequency of LSP oscillation. As a result, the light is absorbed much more efficiently and generates localized and highly amplified electric fields in the proximity of the nanoparticle surface. LSPR occurs when dimensions of metallic materials are much smaller (i.e., 1-100 nm) than the wavelength of incident light (i.e., 300 - 1000 nm). This gives rise to enhanced light scattering and absorption and large local field enhancement, near the NP surface at the resonant condition, which can be controlled by modifying the size, shape, composition, and dielectric properties of the plasmonic nanoparticles and the surrounding environment. Plasmonic nanoparticles are capable of converting the energy of the electromagnetic radiation into heat during the relaxation of excited electrons. Plasmonic nanoparticles may be made of metal, metal oxide, or a mixture of metals and metal oxides. The metals and their oxides include silver, gold, copper, cobalt, nickel, iridium, platinum, ruthenium, rhodium, and palladium, and oxides of these metals. Silver, gold, and copper are preferred metals, and silver and gold are especially preferred metals for use in the invention.

Optical properties of plasmonic nanoparticles can be tuned based on the type of metal, the nanoparticle size and shape, and the surrounding media, to match a specific (pre-determined) visible or near-visible light frequencies with a relatively narrow absorption band.

Plasmonic nanoparticles showing LSPR may be of various sizes and shapes. Sizes can vary from 1 nm up to several hundreds of nm, preferably up to 100 nm and more preferably up to 50 nm. LSPR and optical properties of plasmonic nanoparticles strongly depend on the nanoparticle shape that includes, for example, spheres, hemispheres, rods, fibers, cubes, plates, star-shaped particles, core-shell particles, chains, stars, flowers, and others. Spheres, plates, cubes, star-shaped, and core-shell particles are preferred, and spheres, cubes, plates, and rods are especially preferred shapes.

Plasmonic nanoparticles of one type (material and/or shape) or of several types (materials and/or shapes) may be used in the device of the invention.

The bond of the plasmonic nanoparticles to the polymeric film is based on the electrostatic interaction between the polymeric film and the plasmonic nanoparticles, wherein the (last polyelectrolyte layer of) polymeric film and the plasmonic nanoparticles have opposite surface charges.

The step of applying the plasmonic nanoparticles to the polymeric film is performed by applying a liquid dispersion of plasmonic nanoparticles onto the polymeric film.

Liquid dispersions of plasmonic nanoparticles can be prepared (i) by wet chemical reaction methods such as redox reaction, hydrolysis, precipitation, and/or decomposition reactions, or (ii) by dispersing a solid powder of plasmonic nanoparticles in a liquid media via shaking or using ultrasound, or (iii) by physical condensation or dispersion methods such as laser ablation, electrical disintegration, solvent exchange, excessive cooling or peptidization.
Suitable liquid media to form a dispersion of plasmonic nanoparticles can be polar or non-polar media such as water or organic solvents. Polar media like water or alcohol-based solvents are more preferable media, and water, ethanol and/or isopropanol are the most preferred media.

As an example, dispersion of plasmonic nanoparticles can be synthesized by wet reduction methods using soluble silver, gold, copper, or other metal salts, using reduction by weak or strong inorganic or organic reducing substances such as hydrazine, sodium citrate, saccharides, hydrogen, glycols, polyols or sodium borohydride reducing agents in polar or non-polar environment. In the case of silver or gold plasmonic nanoparticles, various complexing agents (ammonia, sodium or potassium citrate and others), stabilizing and shape modifying agents (surface-activating substances, inorganic ions, synthetic and natural polymers) and various reducing substances (hydrazine, sodium borohydride, saccharides) can be used to produce plasmonic nanoparticles with various sizes and shapes resulting in varied optical properties (LSPR properties). In the case of silver plasmonic nanoparticles, soluble silver salt as a metal precursor, ammonia and potassium citrate as silver complexing and particle stabilizing agents, and strong and weak reducing substance such as sodium borohydride and hydrazine monohydrate in the first and second reduction step, respectively, can be applied to produce silver plasmonic nanoparticles with tunable LSPR covering a large region of spectra from 400 nm to 900 nm.

In order to prepare a layer of plasmonic nanoparticles bonded to the polymeric film, the liquid dispersion of plasmonic nanoparticles is applied on the surface of the support carrying the polymeric film. Volume of the liquid dispersion and concentration of the metal in liquid dispersions of particular plasmonic nanoparticles can typically vary from 0,001 ml to 500 ml and 0,001 µg/ml to 10 mg/ml, preferably from 0,01 ml to 100 ml and 0,01 µg/ml to 1 mg/ml, and more preferably from 0,1 ml to 50 ml and 0,1 µg/ml to 0,1 mg/ml, respectively. After the application, plasmonic nanoparticles dispersed in the liquid dispersion are left to bond electrostatically onto the polymeric film, typically for the period of t 15 min to 5 hours, preferably from 60 min to 3 hours, and more preferably from 90 min to 2 hours. After that, the remaining dispersion of plasmonic nanoparticles is removed, and the surface of the support carrying the polymeric film with the bonded plasmonic nanoparticles is washed by distilled water in order to remove un-bonded plasmonic nanoparticles. Finally, the prepared layer of plasmonic nanoparticles bonded to the polymeric film is dried in ambient or inert conditions at temperatures from 20°C to 50°C. After these procedures, the support with plasmonic nanoparticles bonded to the polymeric film is ready to use for the thermal-microirradiation of cells.

The present invention further provides a method of thermal-microirradiation of cells, comprising the steps of:
- providing the cell culture device of the invention with a cell culture thereon,
- irradiating one or more plasmonic nanoparticles with an electromagnetic irradiation beam (e.g., a laser beam), including absorption frequency of the plasmonic nanoparticles, thereby locally heating the surroundings of the nanoparticle.

The cell culture device can be provided with a cell culture on the surface coated with the polymer matrix layer with embedded plasmonic nanoparticles. The living cells are seeded on the device prior to the irradiation. The cells may also be seeded and grown on the device for various time periods spanning from hours up to weeks. During the growth phase, the cell culture device is immersed in a growth medium. In particular, adherent cell cultures are suitable for growing on the cell culture device.

The irradiation of the nanoparticles is carried out by a source of electromagnetic radiation, wherein the electromagnetic radiation includes radiation having the frequency corresponding to the absorption frequency of the plasmonic nanoparticles. By converting the energy on the corresponding frequency to heat, the plasmonic particles produce heat, which heats their close surroundings locally.

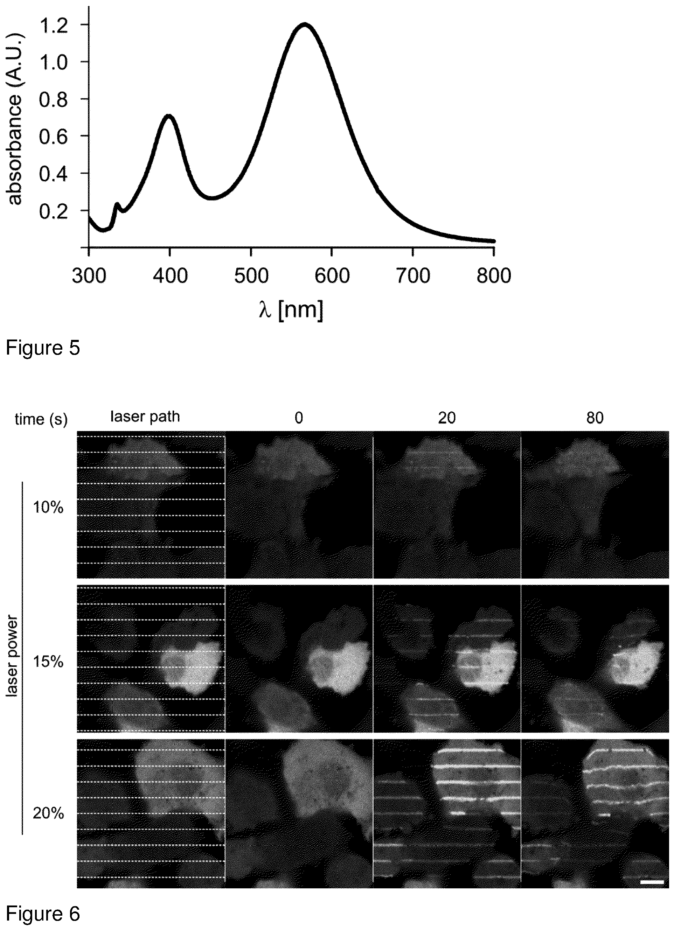

The source of the electromagnetic radiation is preferably a laser having the near-infrared to visible wavelengths, within the range of 300 to 1200 nm. For example for the product described in Example 1 giving an absorption peak of the plasmon layer close to 600nm (as shown in Figure 5) the appropriate lasers include wavelenghts between with 500 - 700 nm, preferable lasers between 550-650nm and more preferable between 560-600nm.

Suitable lasers are, for example, lasers typically used in laser scanning microscopes (LSM), including solid-state lasers and gas lasers. LSMs are specialized fluorescence microscopes used for visualization of microscopic objects such as cells and for various photo manipulations techniques. The photo manipulation techniques allow treating the sample with targeted irradiation. Using the cell culture device of the present invention, a standard LSM can be used for the induction of localized thermal damage.

The focusing of the irradiation beam defines the region of interest (ROI) on the device. The ROI is irradiated by electromagnetic radiation. The ROI may also be referred to as an irradiated area.

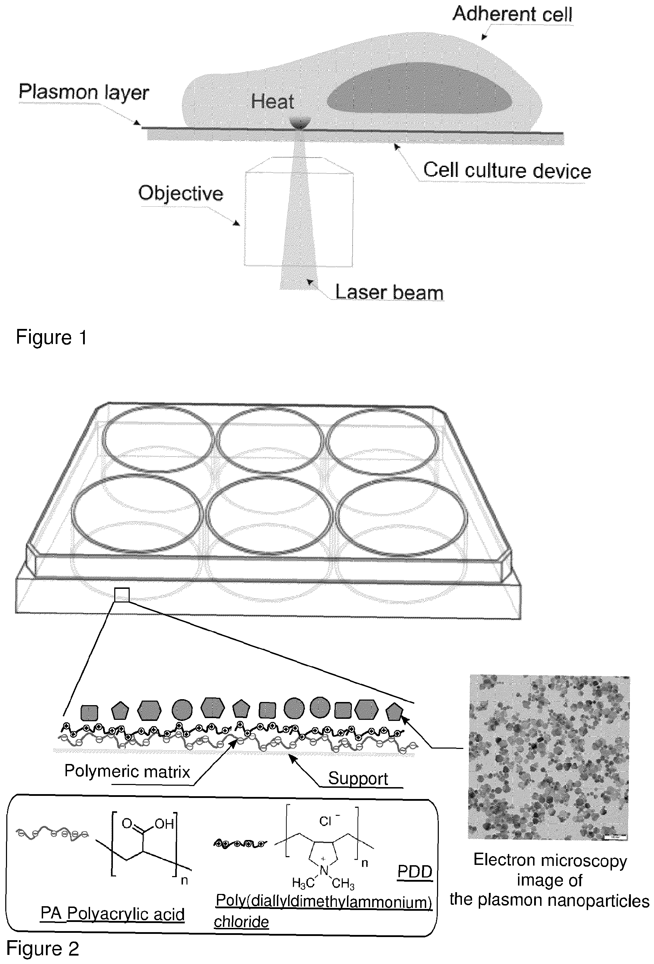

Plasmonic nanoparticle density, laser power, irradiation dwell time, and the number of irradiation cycles control the total amount of heat emitted within the ROI. The cell(s) present in the region of interest are inflicted with local heat damage. The heat generated within the plasmonic nanoparticle(s) is quickly transferred to the adhered or attached cell and leads to the heat damage manifested by rapid denaturation of the heat-affected cellular proteins (see figure 1).

The minimum size of ROI, which can be heat-irradiated, is limited only by the used microscopic optics and by the wavelength of the electromagnetic radiation used to activate plasmonic nanoparticles, as defined by the Abbe's law of limiting resolution, and the spread of thermal dissipation. In practice, the minimal achievable ROI is approx. 200 nm², the maximum achievable size of ROI by single irradiation is limited only by the microscope field of view and can reach up to several mm², for example.

### Brief description of the drawings

Figure 1 shows a schematic representation of the principle of the thermal micro-irradiation of the cells, using the cell culture device of the invention.
Figure 2 is a schematic representation of the cell culture device produced according to Example 4. The TEM image corresponds to the plasmon nanoparticles of Example 1 and was acquired using a JEM 2010 TEM instrument (Jeol, Japan). A droplet of the sample with a silver concentration of 108 mol·dm⁻³ was deposited on a carbon-coated copper grid and dried in a vacuum dryer at 25 °C for 1 hour before TEM acquisition.
Figure 3: Thermal imaging of the laser-irradiated cell culture device with plasmon surface compared to the control cell culture cultivation plate without polymeric film and plasmon surface according to Example 7.
Figure 4: Results of heat damage within a single cell of HSP70-GFP reporter cell line, according to Example 8.
Figure 5: Absorption spectra of plasmon layer deposited on the cultivation surface of the cell culture device, obtained according to Example 9.
Figure 6: Effect of dosing of heat delivery, obtained according to Example 10.

### Examples of Carrying Out the Invention

### Materials and Methods

Silver nitrate (99.9 %, Safina), tetrachloroauric acid trihydrate (Merck Millipore, for analysis), ammonia (25% (w/w) aqueous solution, p.a. Lachema), sodium hydroxide (p.a., Lachema), sodium citrate (p.a., Sigma-Aldrich), sodium borohydride (p.a., Sigma-Aldrich), D(+)-maltose monohydrate (p.a., Riedel-de Haën), and hydrazine monohydrate (65 % (w/w), p.a., Sigma-Aldrich), were used for the preparation of plasmonic NPs without any further purification. Polyacrylic acid (PAA) (M.W. 100.000, p.a., Sigma Aldrich), poly(diallyldimethylammonium chloride) (PDDA) (M.W. 200.000, p.a., Sigma Aldrich), poly(allylamine hydrochloride) (PAH) (M.W. 50.000, p.a., Sigma Aldrich) were used to prepare polymeric film.
The average size and zeta potential (zeta potential expresses the surface charge) of the synthesized plasmonic NPs were determined by the dynamic light scattering and electrophoretic mobility methods, respectively, using a Zetasizer Nano ZS instrument (Malvern, UK). Transmission electron microscopy measurements were conducted using a JEM 2010 instrument (Jeol, Japan). Absorption spectra showing the surface plasmon peak of plasmonic NPs were recorded using a Specord S 600 spectrophotometer (Analytik Jena, Germany).

### Synthesis of plasmonic metal nanoparticles

### Example 1: Synthesis of anisotropic silver nanoparticles (NPs)

Water dispersion of anisotropic silver NPs (108 mg/L) was synthesized by a two-step reduction process, involving partial reduction of the [Ag(NH₃)₂]⁺ complex cation by sodium borohydride in the first step resulting in the formation of the silver nuclei, which were subsequently in the second step grown up by the reduction using weak reducing agent - hydrazine. All the reaction components were, at the laboratory temperature (23 °C), stirred continuously with a magnetic stirrer. Initially, 5 mL of aqueous silver nitrate (0.005 M), 1.25 mL of ammonia solution (0.1 M), 1.25 mL sodium citrate (1% w/w), and 13.425 mL distilled water were added into a 50 mL beaker and stirred while adding reducing agents. The reduction was initiated by the addition 0.075 mL of sodium borohydride (0.001M), which resulted in the reduction of silver complex cation, the formation of small nanoparticles (seeds), and change of the dispersion color to light yellow. Finally, 4 mL of hydrazine solution (0.05 M) was rapidly added into the dispersion of silver seeds under vigorous stirring, resulting in the growth of the seeds into final and stable silver anisotropic nanoparticles followed by a change of the color of the dispersion from light yellow to typical purple. The final reaction concentrations of all the reaction components were as follows: silver nitrate 1·10⁻³ mol·dm⁻³; ammonia 5·10⁻³ mol·dm⁻³; citrate 0.05 % (w/w), sodium borohydride 3·10⁻⁶ mol·dm⁻³, and hydrazine 8·10⁻³ mol·dm⁻³ as reducing agents. The synthesized silver nanoparticles with an average size of 45 nm and negative zeta potential (- 38 mV) show two distinct peaks in absorption spectra with one peak, typical for spherical nanoparticles at 400 nm, and the second one at 600 nm, which is characteristic for the mixture of silver spheres, plates, rods, and triangles. The thus prepared silver nanoparticles were used for nanoparticle deposition on the well surface.
Other plasmonic nanoparticles with different shapes and optical properties (LSPR) can be prepared via the same method, but changing the ratio between citrate and hydrazine, using different amount of citrate varying from 0.25 ml to 4 ml (1% w/w) and adjusting the total volume to 25 ml. By this approach, the silver nanoparticles with plasmon peak within the range of 440 to 725 nm can be synthesized.

### Example 2: Synthesis of spherical silver nanoparticles

Modified Tollens process involving reduction of the complex cation [Ag(NH₃)₂]⁺ by D-maltose was used to synthesize dispersions of spherical silver NPs with a diameter of 28 nm, negative zeta potential (- 28 mV), and silver concentrations of 108 mg L⁻¹. The concentrations of all the reaction components were as follows: silver nitrate 1·10⁻³ mol·dm⁻³; ammonia 5·10⁻³ mol·dm⁻³; sodium hydroxide 9.6·10⁻³ mol·dm⁻³, and D-maltose (as a reducing agent) 1·10⁻² mol·dm⁻³. The reaction system was stirred continuously with a magnetic stirrer, and the reduction was initiated by injecting the reducing agent. After 5 minutes, the reaction was deemed complete, as indicated by the development of the dark yellow color characteristic of silver NP dispersions. All syntheses were conducted at room temperature (approx. 23 °C).

### Example 3: Synthesis of spherical gold nanoparticles

Colloidal dispersions of Au NPs were prepared by the reduction of tetrachloroauric acid with saccharide maltose at the laboratory temperature. For this purpose, 5 ml of 5 mmol·dm⁻³ tetrachloroauric solution was diluted with 15 ml of deionized water. The solutions were vigorously stirred using a magnetic stirrer. The reduction was started by the addition of 10 ml of reducing solution containing maltose at a concentration of 25 mmol·dm⁻³. The formation of the nanoparticles with an average size of 40 nm and negative zeta potential (- 22 mV) was accompanied by a color change from light yellow to violet. The-size distribution of the prepared Au NPs was determined by DLS and UV-Vis spectroscopy after completing the reaction (30 minutes). The UV-Vis spectra were recorded using samples diluted five times due to the high absorbance of the produced dispersions.

### Coating of the substrate by polymeric film and deposition of plasmonic metal nanoparticles on the polymeric film

### Example 4: Coating of a plastic substrate by polymeric film consisting of PAA and PDDA polyions and binding of the silver anisotropic nanoparticles to the polymeric film

Anisotropic silver nanoparticles prepared according to Example 1 were coated onto the bottom of polymer functionalized ibiTreat Ibidi^{™} 24 well plates. For this purpose, wells of the cultivation plates were filled with 1 ml of 1% polyacrylic acid (PAA) aqueous solution followed by 1 ml of 1% poly(diallyldimethylammonium chloride) (PDDA) aqueous solution, each solution was kept in the wells for 2 h, in order to coat them by polymeric film consisting of two layers of oppositely charged polyelectrolytes (PAA and PDDA). Concentrations of the solutions of polyelectrolytes are given in weight % (% w/w). Prior the addition of PDDA into the wells, PAA solution was removed and wells were washed with distilled water. After 4 h of the treatment (2 hours for PAA and 2 hours for PDDA), wells were washed with distilled water again to remove non-bonded polymers and filled with 1 ml of dispersion of silver nanoparticles. Silver nanoparticles were bonded within 45 minutes to polymeric PAA_PDDA film deposited on well surface through the electrostatic interactions between negatively charged silver nanoparticles and positively charged PDDA forming the top layer of the polymeric film. In the end, wells were washed with distilled water in order to remove un-bonded nanoparticles and air-dried.

### Example 5: Coating of a glass substrate by polymeric film consisting of PDDA polyion and binding of silver spherical nanoparticles to the polymeric film

Spherical silver nanoparticles prepared according to Example 2 were coated onto polymer functionalized glass substrate (24 well cultivation plate with glass bottom). Glass substrate was cleaned at first in piranha solution (a mixture of sulfuric acid, water, and hydrogen peroxide) for 30 min and washed with distilled water. After that, the wells of glass substrate were filled with 1 ml of 1% poly(diallyldimethylammonium chloride) (PDDA) aqueous solution for 2 h in order to coat the glass surface by polymeric film consisting of one layer of positively charged polyelectrolyte PDDA. After 2 h of the treatment, wells of the glass substrate were washed with distilled water to remove non-bonded polymer and filled with 1 ml of dispersion of spherical silver nanoparticles. Silver nanoparticles were bonded within 60 minutes to polymeric PDDA film deposited on the glass surface through the electrostatic interactions between negatively charged silver nanoparticles and positively charged PDDA. In the end, wells were washed with distilled water in order to remove un-bonded nanoparticles and air-dried.

### Example 6: Coating of a plastic substrate by polymeric film consisting of PAA and PAH polyions and binding of gold spherical nanoparticles to the polymeric film

Spherical gold nanoparticles prepared according to Example 3 were subsequently coated to onto the bottom of polymer functionalized ibiTreat Ibidi^{™} 24 well plates. For this purpose, wells of the cultivation plates were filled with 1 ml of 1% polyacrylic acid (PAA) aqueous solution followed by 1 ml 2% poly(allylamine hydrochloride) (PAH) aqueous solution, each solution was kept in the wells for 2 h, in order to coat them by polymeric film consisting of two layers of oppositely charged polyelectrolytes (PAA and PAH). Concentrations of the solutions of polyelectrolytes are given in weight % (% w/w). Prior to the addition of PAH into the wells, PAA solution was removed, and wells were washed with distilled water. After 4 h of the treatment (2 hours for PAA and 2 hours for PAH), wells were washed with distilled water to remove non-bonded polymers and filled with 1 ml of dispersion of gold nanoparticles. Gold nanoparticles were bonded within 90 minutes to polymeric PAA_PAH film deposited on well surface through the electrostatic interactions between negatively charged gold nanoparticles and positively charged PAH forming the top layer of the polymeric film. In the end, wells were washed with distilled water in order to remove un-bonded nanoparticles and air-dried.

### Imaging Experiments

### Example 7: Thermal imaging of laser-irradiated cell culture cultivation plate with plasmon surface compared to a control cell culture cultivation plate without plasmon surface

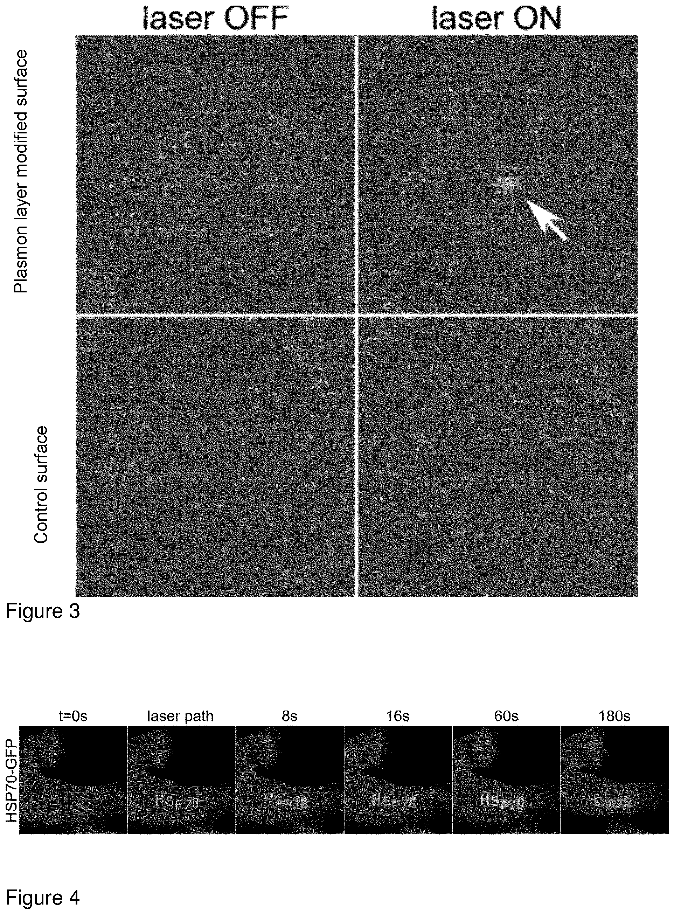

Thermal camera (Therm-App^{®}, Opgal Optronic Industries Ltd.) was placed in an in-house built holder keeping the camera in approx 5cm distance from the top of the cell culture device (24 well plate with the deposited film of plasmon layer on the bottom of the wells according to Example 4). The germanium objective of the thermal camera was manually focused on the bottom of the plate, and the whole assembly was placed inside the Zeiss Axioimager Z.1 platform equipped with LSM780 module for confocal laser scanning microscopy (CLSM). A region of interest (ROI) of the inner surface of the bottom of the well was focused and exposed to the 561nm laser working in the continuous mode while the thermal camera was used to acquire thermograms. An unmodified 24 well plate without the deposited polymeric film and plasmon layer on the bottom of the wells was used as a control. The results are shown in Figure 3. The heat emission after activating laser irradiation is visible as a bright dot (marked by an arrow). In the case of the control plate, no such heat emission is detectable.

### Example 8: Heat damage within a single cell, detected using HSP70-GFP reporter cell line

HSP70 is a protein chaperone known to bind heat-damaged proteins. U2OS human osteosarcoma cell line was used to obtain the HSP70-GFP reporter. Cells were kept in DMEM media (Lonza) supplemented with 10% fetal bovine serum (Thermo Fisher Scientific) and 1% penicillin/streptomycin (Sigma-Aldrich). Piggy Bac transposon system (PB) has been used to generate a stable expression of HSP70-GFP fused protein. The coding sequence was cloned by Gateway recombination technology (Invitrogen, Carlsbad, CA, USA). The full coding sequence of human Hsp70 (HSPA1A) was cloned into PB-EF1a-N-EmGFP-PURO-GW-Dest vector containing an N-terminal GFP tag. 10^E5 cells were transfected using Lipofectamine 3000 (Invitrogen, Carlsbad, CA, USA) with 1800 ng of transposon plasmid and 200 ng of transposase plasmid. Cells were selected in the growth media supplemented with puromycin 5 µg/ml (InvivoGen, San Diego, CA). Flow cytometry and cell sorting (FACS Aria-III, Becton Dickinson) was used to enrich populations with optimal GFP expression.
The cells were seeded into the wells of the cell culture device prepared according to Example 4, 24 hours before the experiment. The device with the seeded cells was placed inside the Zeiss Axioimager Z.1 platform equipped with LSM780 module for confocal laser scanning microscopy (CLSM). The inner surface of the bottom of the well was focused on using Zeiss Alpha Plan-APOCHROMAT 40x water objective. The irradiation laser (561nm) path was defined to form HSP70 letters inside a single cell. The cell device was monitored in the green channel using the same objective in defined time points after the laser irradiation. The obtained images are shown in Figure 4.

### Example 9: Measuring absorption spectra of plasmon layer of cell culture device

The cell culture device prepared according to Example 4 was placed inside Specord S 600 (Analytic Jena, Germany) spectrophotometer. UV-vis spectra of silver nanoparticles were recorded and plotted. The thus obtained absorption spectrum is shown in Figure 5.

### Example 10: Effect of dosing of heat delivery

The HSP70-GFP reporter cell line was generated as described in Example 8. The cells were seeded into the wells of the cell cultivation device manufactured according to example 4, 24 hours before the experiment. The device with the seeded cells was placed inside the Zeiss Axioimager Z.1 platform equipped with LSM780 module for confocal laser scanning microscopy (CLSM). The inner surface of the bottom of the well was focused on using Zeiss Alpha Plan-APOCHROMAT 40x water objective. The irradiation laser path was defined to form co-linear stripes. The plasmon surface was exposed to varying power of the plasmon activation 561nm laser. The cells within the irradiated areas were monitored in time, the results are shown in Figure 6.

## Claims

1. A cell culture device containing a substantially planar support, **characterized in that** it contains:
- a polymeric film containing one or more polyelectrolytes provided on at least one surface of the support;
- a plurality of plasmonic nanoparticles electrostatically bonded to the surface of the polymeric film.

2. The cell culture device according to claim 1, wherein the support is made of glass or transparent plastic, such as polycarbonate, polymethylmethacrylate, polyethylene, polyethylene copolymers, polypropylene, polypropylene copolymers, polystyrene, polystyrene copolymers, styrene methyl methacrylate, poly(styrene-co-acrylonitrile), methyl methacrylate-acrylonitrile-butadiene-styrene, methyl methacrylate-butadiene-styrene, polyacetate, polyamide, polyethyleneterephtalate.

3. The cell culture device according to claim 1 or 2, wherein the polymeric film is formed by a single polyelectrolyte layer, wherein the polyelectrolyte is charged oppositely to the surface of the support or forms hydrogen bonds to the support.

4. The cell culture device according to claim 1 or 2, wherein the polymeric film is formed by two or more polyelectrolyte layers, wherein the first polyelectrolyte layer neighbouring to the support is charged oppositely to the surface of the support or forms hydrogen bonds to the support, and wherein each polyelectrolyte layer is charged oppositely than its neighbouring polyelectrolyte layer(s).

5. The cell culture device according to any one of claims 1-4, wherein the polyelectrolyte layer is a monomolecular layer of a polyelectrolyte.

6. The cell culture device according to any one of claims 1-5, wherein the polyelectrolyte is selected from:
- positively charged polyelectrolytes selected from poly(allylamine hydrochloride), polyethyleneimine, chitosan, poly(diallyldimethylammonium chloride), poly-L-(lysine), and poly(amidoamine); and
- negatively charged polyelectrolytes selected from poly(vinyl sulfate), poly(acrylic acid), poly(beta-amino ester), poly(styrene sulfonate), and poly(methacrylic acid).

7. The cell culture device according to any one of claims 1-6, wherein the plasmonic nanoparticles are made of metal, metal oxide, or a mixture of metals and metal oxides, wherein the metals and their oxides include silver, gold, copper, cobalt, nickel, iridium, platinum, ruthenium, rhodium, and palladium, and oxides of these metals.

8. The cell culture device according to any one of claims 1-7, wherein the plasmonic nanoparticles have a shape selected from spheres, hemispheres, rods, fibers, cubes, plates, star-shaped particles, core-shell particles, chains, stars, flowers.

9. A method of preparation of the cell culture device according to any one of claims 1-8, said method comprising the steps of:
- providing a substantially planar support;
- applying a polymeric film containing one or more polyelectrolytes on at least one surface of the support;
- applying plasmonic nanoparticles onto the surface of the polymeric film.

10. The method according to claim 9, wherein the step of applying a polymeric film containing one or more polyelectrolytes on at least one surface of the support is carried out by applying a solution of a polyelectrolyte on the surface of the support, allowing the polyelectrolyte to bond electrostatically or through hydrogen bond or adsorb, removing the remaining polyelectrolyte solution and washing the surface, and optionally repeating these steps with solutions of further polyelectrolytes.

11. The method according to claim 9 or 10, wherein the step of applying the plasmonic nanoparticles to the polymeric film is performed by applying a liquid dispersion of plasmonic nanoparticles onto the polymeric film, wherein the liquid dispersion of plasmonic nanoparticles is prepared
(i) by wet chemical reaction methods such as redox reaction, hydrolysis, precipitation, and/or decomposition reactions, or
(ii) by dispersing a solid powder of plasmonic nanoparticles in a liquid medium via shaking or using ultrasound, or
(iii) by physical condensation or dispersion methods such as laser ablation, electrical disintegration, solvent exchange, excessive cooling or peptidization;
and allowing the plasmonic nanoparticles dispersed in the liquid dispersion to bond electrostatically onto the polymeric film, washing away the un-bonded plasmonic nanoparticles, and drying the prepared layer of plasmonic nanopartcles at a temperature within the range of 20°C to 50°C.

12. A method of thermal micro-irradiation of cells, comprising the steps of:
- providing the cell culture device according to any one of claims 1-8 with a cell culture thereon,
- irradiating one or more plasmonic nanoparticles with an electromagnetic irradiation beam, including absorption frequency of the plasmonic nanoparticles, thereby locally heating the nanoparticle and the surroundings of the nanoparticle.

13. The method according to claim 12, wherein the source of the electromagnetic irradiation beam is preferably a laser having wavelength within the range of 300 to 1200 nm.
